Europäisches Patentamt

European Patent Office

Office européen des brevets

(11) Veröffentlichungsnummer: **0 433 927 B1**

# (12) EUROPÄISCHE PATENTSCHRIFT

(45) Veröffentlichungstag der Patentschrift: **20.09.95**

(21) Anmeldenummer: **90124304.8**

(22) Anmeldetag: **15.12.90**

(51) Int. Cl.6: **G01N 33/68**, G01N 33/86, C07K 14/00, G01N 33/92, G01N 33/53, G01N 33/74

(54) **Mittel zur Verbesserung der Wiederfindung von Annexinen.**

(30) Priorität: **20.12.89 DE 3942081**

(43) Veröffentlichungstag der Anmeldung:
**26.06.91 Patentblatt 91/26**

(45) Bekanntmachung des Hinweises auf die
Patenterteilung:
**20.09.95 Patentblatt 95/38**

(84) Benannte Vertragsstaaten:
**AT BE CH DE ES FR GB IT LI LU NL SE**

(56) Entgegenhaltungen:
**EP-A- 0 040 799**

**ONCODEVELOPMENT IN BIOLOGY AND MEDICINE Bd. 2, Nr. 3, 1981, BRD Seiten 141 - 154 H. BOHN ET AL. 'New placental proteins and their potential diagnostic significance as tumor markers.'**

**MEDICAL MICROBIOLOGY AND IMMUNOLOGY Bd. 180, Nr. 3, 1991, BRD Seiten 109 - 126 J. ROEMISCH ET AL. 'Annexins calcium-binding proteins of multi-functional importance.'**

(73) Patentinhaber: **BEHRINGWERKE Aktiengesellschaft**
**Postfach 1140**
**D-35001 Marburg (DE)**

(72) Erfinder: **Römisch, Jürgen, Dr.**
**Am Kähnelplatz 6**
**W-3550 Marburg (DE)**
Erfinder: **Auerbach, Bernhard, Dr.**
**Holderstrauch 7**
**W-3550 Marburg (DE)**
Erfinder: **Pelzer, Hermann, Dr.**
**Calvinstrasse 3**
**W-3550 Marburg (DE)**

(74) Vertreter: **Fischer, Hans-Jürgen, Dr. et al**
**Hoechst AG**
**Patent- und Lizenzabteilung**
**Gebäude K 801**
**D-65926 Frankturt am Main (DE)**

## Beschreibung

Die Erfindung betrifft ein Mittel zur Stabilisierung der funktionellen Aktivität von Annexinen sowie geeignete Komponenten zum Sammeln von Körperflüssigkeiten für deren quantitative Bestimmung.

Als Annexine oder auch Lipocortine oder Calpactine werden u.a. die Proteine PP4, PP4-X, PAP III, p68, Anchorin und Lipocortin I und II bezeichnet. Ihre physiologische Bedeutung ist noch nicht vollständig geklärt.

Annexine wurden in vielen Spezies, humanen Organen und unterschiedlichen Zell-Typen nachgewiesen. Sie stellen nach dem bisherigen Wissenstand ein großes antiinflammatorisches bzw. antikoagulatorisches Potential dar, jedoch sind diese Proteine in Körperflüssigkeiten, insbesonders im Blut, nur in sehr geringen Konzentrationen vorhanden (1-5 ng/ml). Wie sich aber am Beispiel des PP4 zeigte, sind sie bei bestimmten Krankheiten oftmals in stark erhöhten Konzentrationen in Körperflüssigkeiten, beispielsweise in Blut-Plasma, nachweisbar und können somit als Marker für eine Früherkennung oder für die Verfolgung eines Krankheitsverlaufes von erheblicher klinischer Relevanz sein (Gocze, P.M. et al. (1988) Med. Sci. Res. 16, 407-408). Dies gilt in gleichem Maße für die Proteine PP4-X, PAP III, p68, Anchorin sowie Lipocortin I und II. Die quantitative Bestimmung der Proteine kann mittels empfindlicher Nachweismethoden, wie z. B. RIA oder ELISA erfolgen.

Entscheidend für die Aussagekraft eines Testes ist der Normalbereich, welcher die Konzentration des betreffenden Analyten in Körperflüssigkeiten eines Referenzkollektives zusammengesetzt aus gesunden Probanden, angibt im Vergleich zum pathologischen Bereich. Daraus ergibt sich eine Obergrenze des Normalbereiches, die auch als cut-off bezeichnet wird. Bei solch empfindlichen Testmethoden wie RIA oder ELISA ist u. a. auch die Gewinnung der Körperflüssigkeiten von großer Bedeutung, da z.B. beim Sammeln von Blut dessen zelluläre Bestandteile in Mitleidenschaft gezogen und dadurch die Test-Ergebnisse verfälscht werden könnten. Oft können unerwünschte Nebeneffekte, wie ein zu hoher "Background" bei der späteren Bestimmung durch sorgfältige Venenpunktion und schnelles Mischen der zu sammelnden Flüssigkeit mit für die zu bestimmende Substanz schützenden Zusätzen vermieden werden. Eine bekannte Form eines Mittels zur Blutabnahme ist Citrat, das die Gerinnung durch Komplexierung von Kalzium-Ionen verhindert.

Ein Nachteil des Citrat als Mittel zur Probenstabilisierung ist jedoch, daß die durch Beimischung dieses Mittels gewonnenen Proben nicht zur Quantifizierung der Annexine geeignet sind (Tabelle 1). Die am Beispiel des PP4 durchgeführten Tests zeigten, daß die durch ELISA bestimmten PP4-Konzentrationen in Plasmen, die aus Vollblut, das mit Citrat als Antikoagulans versetzt war, von 10 gesunden Spendern stammten, zwischen 2 und 8 ng/ml lagen und für einen solchen Test nicht tolerierbar hohe Schwankungen aufwiesen. Auch EDTA-Plasmen erbrachten keine befriedigenden Ergebnisse, da auch mit diesem Chelatbildner die Festlegung einer akzeptablen Obergrenze unmöglich war und große Schwankungen bei "Normalspendern" auftraten.

Der vorliegenden Erfindung lag daher die Aufgabe zugrunde, ein Mittel zur Stabilisierung der funktionellen Aktivität der Annexine zu finden, das es ermöglicht, eine zuverlässige Quantifizierung der Annexine in Körperflüssigkeiten durchzuführen.

Erfindungsgemäß wird nun zur Probennahme ein Mittel gemäß Anspruch 1 eingesetzt.

Die Festlegung eines Referenzbereiches sowie ein ausreichend niedriger "cut-off" für die Bestimmung der Annexine wird dadurch ermöglicht.

Die Blutplättchenaggregationsinhibitoren werden im Testansatz in Konzentrationen bevorzugterweise von 2 - 200 mmol/l, ganz besonders bevorzugterweise von 5 - 50 mmol/l eingesetzt.

Von Blutplättchenaggregationsinhibitoren ist bekannt, daß sie mehr oder weniger gut die Aggregation von Blutplättchen und die Ausschüttung z.B. der Plättchenfaktor (PF4) enthaltenden α-Granulae inhibieren (Prowse, C. et al. (1982) Thromb. Res. 25, 219-227). Ohne damit eine Wirkungsweise festlegen zu wollen, kann gesagt werden, daß die oben beschriebene Erfindung jedoch nicht auf einer Inhibition der α-Granulae-Ausschüttung beruht, da trotz stark schwankender PF4-Werte keine Korrelation zu den ermittelten PP4-Werten erkennbar ist (Tabelle 1). Optimale PP4-"Normalwerte" werden nur mit dem erfindungsgemäßen Mittel erzielt.

In einer Verfahrensweise wird eine Körperflüssigkeit, beispielsweise Blut, mit einem Mittel, das in einer solchen Konzentration vorliegt, daß das Gemisch aus Mittel und Probe pro Liter bevorzugterweise 2 - 200 mmol, ganz besonders bevorzugterweise 5 - 50 mmol eines Chelatbildners, beispielsweise EDTA, EGTA oder ein Salz der Citronensäure oder der Oxalsäure, enthält bevorzugterweise 500 - 50 000 Einheiten eines Antikoagulans wie z.B. Heparin, Hirudin, Chondroitinsulfat, Dermatansulfat oder ein Pentosanpolysulfat, und 2-200 mmol einer oder mehrerer der o.g. Blutplättchenaggregationsinhibitoren enthält, während oder kurz nach der Abnahme vermischt und die zellulären Bestandteile, falls vorhanden, von der Flüssigkeit getrennt.

Sowohl die Chelatbildner und die Antikoagulantien als auch die Zusatzstoffe können jeweils allein, kombiniert oder zusammen in der beschriebenen Kombination verwendet werden. Diese Verfahrensweise ist sowohl für die quantitative Bestimmung von PP4 als auch der anderen Annexine PP4-X, PAP III, p68, Anchorin, Lipocortin I und II geeignet.

In einer bevorzugten Verfahrensweise werden neun Teile einer Körperflüssigkeit mit einem Teil einer Lösung, die 2 - 200 mmol EDTA, 5000 - 50.000 Einheiten Heparin und 5 - 50 mol mindestens einer der folgenden Substanzen Chloroquin, Hydroxychloroquin und Quinacrin enthält, vermischt und die zellulären Bestandteile, falls vorhanden, von der Flüssigkeit getrennt.

Antikoagulantien sind dem Fachmann an sich bekannte Verbindungen wie z. B. Hirudin, Heparin, Dermatansulfat, oder Pentosansulfate, bevorzugt sind dabei im besonderen Heparin und Hirudin. Antikoagulatorische Wirkung haben auch die hier als Chelatbildner bezeichneten, an sich dem Fachmann bekannten Substanzen, wie z. B. Citrate, Oxalate, EDTA oder EGTA, wobei bevorzugt sind EDTA oder EGTA.

Die optimalen Konzentrationen sind dem Fachmann an sich bekannt.

Ganz bevorzugt sind Mittel wie in dem "Beispiel 1: Medium C" beschrieben.

Die Erfindung soll durch die folgenden Beispiele erläutert werden:

Beispiel 1:

Probenentnahme

Am Oberarm gesunder Blutspender wurde ein Stauungsdruck von ca. 40 mm Hg erzeugt und nach exakter Venenpunktion mit einer "Butterfly"-Kanüle wurden 4,5 ml Vollblut in eine Einmalspritze aufgezogen, in der 0,5 ml Entnahmemedium vorgelegt waren. Als Entnahmemedien wurden folgende Lösungen verwendet:

Medium A:    110 mmol/l Trinatriumcitrat-Lösung

Medium B:    27 mmol/l EDTA-Lösung mit Zusatz von 0,9% NaCl, pH 7,4

Medium C:    134 mmol/l EDTA-Lösung mit Zusatz von 20 000 Einheiten Heparin/l und 16 mmol/l Hydroxychloroquinsulfat, pH 7,4

Die Proben wurden anschließend sofort in Polystyrolröhrchen überführt und bis zu ihrer Weiterverarbeitung mindestens 30 min aber maximal 2 Stunden bei +2 bis +8°C gelagert.

Die gekühlten Proben wurden bei 2000xg für 15 min zentrifugiert und der Plasmaüberstand weitere 15 min bei 8000xg. Plasmaüberstände wurden zur Messung im PP4-Enzymimmunoassay eingesetzt. Die mit Entnahmemedium C versehenen Proben wurden zusätzlich im PF4-Immunoassay getestet.

PF4- und PP4-Immunoassays

Die PF4-Konzentration der mit dem Entnahmemedium C versehenen Proben wurde mit dem Enzygnost-Plättchenfaktor 4 -Enzymimmunoassay (OULV, Behringwerke) entsprechend der Testvorschrift bestimmt. Zur Messung der PP4-Konzentration wurden die weiter unten beschriebenen testspezifischen Komponenten sowie Reagenzien aus kommerziell erhältlichen Testkits der Behringwerke verwendet: Je 100 $\mu$l Standard (0,3 bis 10 $\mu$g/l; Ch.-Nr. 268907, BW) bzw. 100 $\mu$l mit dem TAT-Probenpuffer (OURG, BW) 1:2 oder 1:11 verdünnte Plasmaproben wurden in die Vertiefungen von Mikrotitrationsplatten (Nunc), die mit polyklonalen Anti-PP4-Antikörpern (v. Kaninchen, Ch.-Nr. 278905, BW) beschichtet waren, pipettiert und 2 Stunden bei 37°C inkubiert. Nach dreimaligem Waschen mit dem verdünnten Enzygnost-Waschpuffer (OSEW), BW) wurden in die einzelnen Vertiefungen je 100 $\mu$l einer Anti-PP4-Antikörper/Peroxidase-Konjugationslösung (Ch.-Nr. 308808, BW) eingefüllt. Der folgende zweistündige Inkubationsschritt bei 37°C wurde mit einem dreimaligen Waschzyklus abgeschlossen. Für den dritten Inkubationsschritt (Raumtemperatur) wurden anschließend je 100 $\mu$l einer Puffer/Substrat-Chromogenlösung (TMB; OUVG/OUVF, BW) in die Vertiefungen pipettiert und die Enzymreaktion nach 30 min mit Enzygnost-Stopplösung (OSFA, BW) beendet. Die Extinktion der Standards und der Proben wurde bei 450 nm ermittelt und die PP4-Konzentration anhand der erstellten Standardkurve unter Einbeziehung des Verdünnungsfaktors berechnet.

Ergebnis

Die niedrigsten PP4-Konzentrationen wurden in den mit den Entnahmemedium C versehenen Plasmaproben gemessen. Beim Medium B und vor allem beim Medium A wurden deutliche höhere, von Proband zu Proband stark unterschiedliche PP4-Werte gefunden (Tab. 1).

Tab. 1:    PF4- und PP4-Meßwerte in verschiedenen Plasmaproben (von 10 gesunden Blutspendern)

Tabelle 1

| Entnahmemedium: | PF4 ($\mu$g/l) C | | PP4 ($\mu$g/l) A | B | C |
|---|---|---|---|---|---|
| Blutspender: | | | | | |
| 1 | 3.7 | | 5.1 | 1.0 | 1.9 |
| 2 | 3.5 | | 4.5 | 1.5 | 0.7 |
| 3 | 8.7 | | 7.6 | 5.3 | 1.2 |
| 4 | 2.4 | | 4.4 | 0.8 | 1.0 |
| 5 | 3.2 | | 2.0 | 1.9 | 0.7 |
| 6 | 6.4 | | 2.8 | 1.8 | 0.7 |
| 7 | 3.9 | | 8.0 | 1.7 | 0.9 |
| 8 | 2.5 | | 3.3 | 1.0 | 0.7 |
| 9 | 4.2 | | 2.4 | 1.4 | 1.0 |
| 10 | 4.4 | | 2.0 | 0.9 | 1.1 |

Beispiel 2

Um auszuschließen, daß die Entnahmemedien die Wiederfindung von PP4 im Enzymimmunoassay beeinträchtigen, wurden bei der 1:11 Verdünnung der Plasmaproben mit dem Probenpuffer gleichzeitig 5,5 ng Standard-PP4 zugesetzt.

Die PP4-Wiederfindungsrate war bei allen Entnahmemedien annähernd gleich (Tab. 2), sodaß ausschließlich das Verfahren und die Wahl des geeigneten Entnahmemediums bei der Blutabnahme einen erhöhten PP4-Level bei gesunden Normalpersonen bedingt.

Tab. 2: Wiederfindungsrate von PP4 (%) in verschiedenen Plasmen (n = 10)

Tabelle 2

| | |
|---|---|
| Medium A: | 87,0 +/- 9,2 % |
| Medium B: | 87,5 +/- 7,5 % |
| Medium C: | 87,8 +/- 6,7 % |

**Patentansprüche**
**Patentansprüche für folgende Vertragsstaaten : DE, GB, FR, IT, NL, SE, LI, CH, BE, AT, LU**

1.  Mittel zum Stabilisieren der funktionellen Aktivität von Annexinen, das in wässriger Lösung enthält:
    0,2 - 200 mmol/l eines oder mehrerer Blutplättchenaggregationsinhibitoren,
    0,2 - 500 mmol/l eines Chelatbildners und 100 - 100 000 Einheiten eines Antikoagulanz,
    wobei die Blutplättchenaggregationsinhibitoren ausgewählt werden aus der folgendenden Gruppe von Substanzen: Chloroquin, Hydroxychloroquin, Quinacrin, Camoquin, Dibucain, Lidocain, Procain, Tetracain, Chlorpromazin, Trifluoperazin, Reserpin, Acetylsalicylsäure, Indomethacin, Proquazon, Prenylamin-Lactat, Perhexilin-Maleat, Nifedipin, Verapamil und Pentoxifyllin.

2. Mittel gemäß Anspruch 1 dadurch gekennzeichnet, daß der oder die Blutplättchenaggregationsinhibitoren in einer Gesamtkonzentration von 2 - 200 mmol/l, vorzugsweise 5 - 50 mmol/l in der gebrauchsfertigen Lösung enthalten sind.

3. Mittel gemäß Anspruch 1 oder 2 dadurch gekennzeichnet, daß als Blutplättchenaggregationsinhibitor Chloroquin, Hydroxychloroquin, Quinacrin oder ein Gemisch aus mindestens zwei dieser Substanzen eingesetzt wird.

4. Mittel nach mindestens einem der Ansprüche 1 - 3 dadurch gekennzeichnet, daß als Antikoagulans Hirudin, Heparin, Dermatansulfat, Chondroitinsulfat oder ein Pentosanpolysulfat in einer Konzentration von 100 - 100 000 Einheiten pro Liter oder eine Kombination verwendet wird, vorzugsweise aber 5000 - 50000 Einheiten Heparin pro Liter der gebrauchsfertigen Lösung.

5. Mittel nach mindestens einem der Ansprüche 1 - 4 dadurch gekennzeichnet, daß als Chelatbildner ein Salz der Citronensäure oder der Oxalsäure oder EDTA, EGTA oder eine Kombination dieser Substanzen in einer Konzentration von 0,2 - 500 mmol/l verwendet wird, vorzugsweise 2 - 200 mmol/l EDTA.

6. Verfahren zum Nachweis der funktionellen Aktivität der Proteine PP4, PP4-X, PAP III, p68, Anchorin und Lipocortin I und II, unter Verwendung von spezifischen Bindungspartnern dadurch gekennzeichnet, daß das Mittel nach mindestens einem der Ansprüche 1 - 5 verwendet wird.

7. Verfahren nach Anspruch 6 dadurch gekennzeichnet, daß als spezifische Bindungspartner Antikörper verwendet werden und die Konzentration der Proteine beispielsweise mittels EIA, RIA, CIA oder FIA bestimmt wird.

8. Verfahren nach Anspruch 6 oder 7 dadurch gekennzeichnet, daß eine Blutprobe in einem Gefäß aufgefangen wird, in dem ein solches Volumen einer mehrfach konzentrierten Lösung des Mittels gemäß Anspruch 1 vorgelegt wird, daß nach der Blutzugabe die gewünschte Konzentration der Wirkstoffe des Mittels erreicht wird und dann in dieser Mischung oder einer Probe davon die funktionelle Aktivität von Annexinen bestimmt wird.

**Patentansprüche für folgenden Vertragsstaat : ES**

1. Verfahren zur Herstellung eines Mittels zum Stabilisieren der funktionellen Aktivität von Annexinen, dadurch gekennzeichnet, daß in wässriger Lösung
0,2 - 200 mmol/l eines oder mehrerer Blutplättchenaggregationsinhibitoren,
0,2 - 500 mmol/l eines Chelatbildners und 100 - 100 000 Einheiten eines Antikoagulanz, vermischt werden,
wobei die Blutplättchenaggregationsinhibitoren ausgewählt werden aus der folgendenden Gruppe von Substanzen: Chloroquin, Hydroxychloroquin, Quinacrin, Camoquin, Dibucain, Licocain, Procain, Tetracain, Chlorpromazin, Trifluoperazin, Reserpin, Acetylsalicylsäure, Indomethacin, Proquazon, Prenylamin-Lactat, Perhexilin-Maleat, Nifedipin, Verapamil und Pentoxifyllin.

2. Verfahren gemäß Anspruch 1 dadurch gekennzeichnet, daß der oder die Blutplättchenaggregationsinhibitoren in einer Gesamtkonzentration von 2 - 200 mmol/l, vorzugsweise 5 - 50 mmol/l in der gebrauchsfertigen Lösung enthalten sind.

3. Verfahren gemäß Anspruch 1 und 2 dadurch gekennzeichnet, daß als Blutplättchenaggregationsinhibitor Chloroquin, Hydroxychloroquin, Quinacrin oder ein Gemisch aus mindestens zwei dieser Substanzen eingesetzt wird.

4. Verfahren nach mindestens einem der Ansprüche 1 - 3 dadurch gekennzeichnet, daß als Antikoagulans Hirudin, Heparin, Dermatansulfat, Chondroitinsulfat oder ein Pentosanpolysulfat in einer Konzentration von 100 - 100 000 Einheiten pro Liter oder eine Kombination verwendet wird, vorzugsweise aber 5000 - 50000 Einheiten Heparin pro Liter der gebrauchsfertigen Lösung.

5. Verfahren nach mindestens einem der Ansprüche 1 - 4 dadurch gekennzeichnet, daß als Chelatbildner ein Salz der Citronensäure oder der Oxalsäure oder EDTA, EGTA oder eine Kombination dieser Substanzen in einer Konzentration von 0,2 - 500 mmol/l verwendet wird, vorzugsweise 2 - 200 mmol/l EDTA.

6. Verfahren zum Nachweis der funktionellen Aktivität der Proteine PP4, PP4-X, PAP III, p68, Anchorin und Lipocortin I und II, unter Verwendung von spezifischen Bindungspartnern dadurch gekennzeichnet, daß das nach mindestens einem der Ansprüche 1 - 5 hergestellte Mittel verwendet wird.

7. Verfahren nach Anspruch 6 dadurch gekennzeichnet, daß als spezifische Bindungspartner Antikörper verwendet werden und die Konzentration der Proteine beispielsweise mittels EIA, RIA, CIA oder FIA bestimmt wird.

8. Verfahren nach Anspruch 6 oder 7 dadurch gekennzeichnet, daß eine Blutprobe in einem Gefäß aufgefangen wird, in dem ein solches Volumen einer mehrfach konzentrierten Lösung des Mittels hergestellt gemäß Anspruch 1 vorgelegt wird, daß nach der Blutzugabe die gewünschte Konzentration der Wirkstoffe des Mittels erreicht wird und dann in dieser Mischung oder einer Probe davon die funktionelle Aktivität von Annexinen bestimmt wird.

**Claims**
**Claims for the following Contracting States : DE, GB, FR, IT, NL, SE, LI, CH, BE, AT, LU**

1. An agent for stabilizing the functional activity of annexins which contains in aqueous solution: 0.2 - 200 mmol/l of one or more platelet aggregation inhibitors,
0.2 - 500 mmol/l of a chelating agent and 100 - 100,000 units of an anticoagulant,
the platelet aggregation inhibitors being selected from the following group of substances: chloroquine, hydroxychloroquine, quinacrine, camoquin, dibucaine, lidocaine, procaine, tetracaine, chlorpromazine, trifluoperazine, reserpine, acetylsalicylic acid, indomethacin, proquazone, prenylamine lactate, perhexiline maleate, nifedipine, verapamil and pentoxifylline.

2. An agent as claimed in claim 1 which contains the platelet aggregation inhibitor(s) in a total concentration of 2 - 200 mmol/l, preferably 5 - 50 mmol/l, in the solution ready for use.

3. An agent as claimed in claim 1 or 2, wherein chloroquine, hydroxychloroquine, quinacrine or a mixture of at least two of these substances is used as platelet aggregation inhibitor.

4. An agent as claimed in at least one of claims 1-3, wherein hirudin, heparin, dermatan sulfate, chondroitin sulfate or a pentosan polysulfate in a concentration of 100 - 100,000 units per liter, or a combination, but preferably 5000 - 50,000 units of heparin per liter of the solution ready for use is used as anticoagulant.

5. An agent as claimed in at least one of claims 1-4, wherein a salt of citric acid or of oxalic acid or EDTA, EGTA or a combination of these substances in a concentration of 0.2 - 500 mmol/l is used, preferably 2 - 200 mmol/l EDTA, as chelating agent.

6. A process for detection of the functional activity of the proteins PP4, PP4-X, PAP III, p68, anchoring and lipocortin I and II using specific binding partners, which comprises the use of the agent as claimed in at least one of claims 1 - 5.

7. The process as claimed in claim 6, wherein antibodies are used as specific binding partners, and the concentration of the proteins is determined for example by means of EIA, RIA, CIA or FIA.

8. The process as claimed in claim 6 or 7, wherein a blood sample is collected in a vessel into which such a volume of a several-fold concentrated solution of the agent as claimed in claim 1 is initially introduced that, after the addition of blood, the desired concentration of the active substances of the agent is reached and then the functional activity of annexins is determined in this mixture or a sample

thereof.

**Claims for the following Contracting State : ES**

1. A process for preparing an agent for stabilizing the functional activity of annexins, which comprises 0.2 - 200 mmol/l of one or more platelet aggregation inhibitors, 0.2 - 500 mmol/l of a chelating agent and 100 - 100,000 units of an anticoagulant being mixed together in aqueous solution, the platelet aggregation inhibitors being selected from the following group of substances: chloroquine, hydroxychloroquine, quinacrine, camoquin, dibucaine, lidocaine, procaine, tetracaine, chlorpromazine, trifluoperazine, reserpine, acetylsalicylic acid, indomethacin, proquazone, prenylamine lactate, perhexiline maleate, nifedipine, verapamil and pentoxifylline.

2. The process as claimed in claim 1 which contains the platelet aggregation inhibitor(s) in a total concentration of 2 - 200 mmol/l, preferably 5 - 50 mmol/l, in the solution ready for use.

3. The process as claimed in claim 1 and 2, wherein chloroquine, hydroxychloroquine, quinacrine or a mixture of at least two of these substances is used as platelet aggregation inhibitor.

4. The process as claimed in at least one of claims 1-3, wherein hirudin, heparin, dermatan sulfate, chondroitin sulfate or a pentosan polysulfate in a concentration of 100 - 100,000 units per liter, or a combination, but preferably 5000 - 50,000 units of heparin per liter of the solution ready for use is used as anticoagulant.

5. The process as claimed in at least one of claims 1-4, wherein a salt of citric acid or of oxalic acid or EDTA, EGTA or a combination of these substances in a concentration of 0.2 - 500 mmol/l, preferably 2 - 200 mmol/l EDTA, is used as chelating agent.

6. A process for the detection of the functional activity of the proteins PP4, PP4-X, PAP III, p68, anchorin and lipocortin I and II using specific binding partners, which comprises the use of the agent prepared as claimed in at least one of claims 1-5.

7. The process as claimed in claim 6, wherein antibodies are used as specific binding partners, and the concentration of the proteins is determined for example by means of EIA, RIA, CIA or FIA.

8. The process as claimed in claim 6 or 7, wherein a blood sample is collected in a vessel into which such a volume of a several-fold concentrated solution of the agent prepared as claimed in claim 1 is initially introduced that, after the addition of blood, the desired concentration of the active substances of the agent is reached and then the functional activity of annexins is determined in this mixture or a sample thereof.

**Revendications**
**Revendications pour les Etats contractants suivants : DE, GB, FR, IT, NL, SE, LI, CH, BE, AT, LU**

1. Agent pour la stabilisation de l'activité fonctionnelle d'annexines, qui contient en solution aqueuse :
   0,2 - 200 mmoles/l d'un ou plusieurs inhibiteurs de l'agrégation plaquettaire ;
   0,5 - 500 mmoles/l d'un agent chélateur ; et
   100 - 100 000 unités d'un anticoagulant ; les inhibiteurs de l'agrégation plaquettaire étant choisis dans le groupe suivant de substances :
   la chloroquine, l'hydroxychloroquine, la quinacrine, la camoquine, la dibucaïne, la lidocaïne, la procaïne, la tétracaïne, la chlorpromazine, la trifluopérazine, la réserpine, l'acide acétylsalicylique, l'indométacine, la poquazone, le lactate de prénylamine, le maléate de perhexiline, la niféfipine, le vérampil et la pentoxifylline.

2. Agent selon la revendication 1, caractérisé en ce que l'inhibiteur ou les inhibiteurs de l'agrégation plaquettaire est(sont) contenu(s) à une concentration totale de 2 - 200 mmoles/l, de préférence de 5 - 50 mmoles/l dans la solution prête à l'emploi.

**3.** Agent selon la revendication 1 ou 2, caractérisé en ce que, comme inhibiteur de l'agrégation plaquettaire, on utilise la chloroquine, l'hydroxychloroquine, la quinacrine ou un mélange d'au moins deux de ces substances.

**4.** Agent selon au moins l'une des revendications 1 à 3, caractérisé en ce que, comme anticoagulant, on utilise l'hirudine, l'héparine, le dermatane-sulfate, le chondroïtine-sulfate ou un pentosane-polysulfate, à une concentration de 100 - 100 000 unités par litre, ou une association, mais de préférence 5 000 - 50 000 unités d'héparine par litre de la solution prête à l'emploi.

**5.** Agent selon au moins l'une des revendications 1 à 4, caractérisé en ce que, comme agent chélateur, on utilise un sel de l'acide citrique ou de l'acide oxalique, ou l'EDTA, l'EGTA ou une association de ces substances, à une concentration de 0,2 - 500 mmoles/l, de préférence 2 - 200 mmoles/l d'EDTA.

**6.** Procédé pour la mise en évidence de l'activité fonctionnelle des protéines PP4, PPA-X, PAP III, p68, anchorine et lipocortines I et II, au moyen de partenaires de liaison spécifiques, caractérisé en ce que l'on utilise l'agent selon au moins l'une des revendications 1 à 5.

**7.** Procédé selon la revendication 6, caractérisé en ce que, comme partenaires de liaison spécifiques, on utilise des anticorps, et la concentration des protéines est déterminée par exemple au moyen d'un essai EIA, RIA, CIA ou FIA.

**8.** Procédé selon la revendication 6 ou 7, caractérisé en ce que l'on recueille un échantillon de sang dans un récipient dans lequel est disposé au préalable un volume d'une solution plusieurs fois concentrée de l'agent selon la revendication 1, tel qu'après l'addition de sang la concentration désirée de la substance active de l'agent est atteinte, et on détermine ensuite dans ce mélange, ou dans un échantillon de celui-ci, l'activité fonctionnelle d'annexines.

**Revendications pour l'Etat contractant suivant : ES**

**1.** Procédé pour la préparation d'un agent pour la stabilisation de l'activité fonctionnelle d'annexines, caractérisé en ce que l'on mélange en solution aqueuse :
0,2 - 200 mmoles/l d'un ou plusieurs inhibiteurs de l'agrégation plaquettaire ;
0,5 - 500 mmoles/l d'un agent chélateur ; et
100 - 100 000 unités d'un anticoagulant ; les inhibiteurs de l'agrégation plaquettaire étant choisis dans le groupe suivant de substances :
la chloroquine, l'hydroxychloroquine, la quinacrine, la camoquine, la dibucaïne, la lidocaïne, la procaïne, la tétracaïne, la chlorpromazine, la trifluopérazine, la réserpine, l'acide acétylsalicylique, l'indométacine, la poquazone, le lactate de prénylamine, le maléate de perhexiline, la nifédipine, le vérampil et la pentoxifylline.

**2.** Procédé selon la revendication 1, caractérisé en ce que l'inhibiteur ou les inhibiteurs de l'agrégation plaquettaire est(sont) contenu(s) à une concentration totale de 2 - 200 mmoles/l, de préférence de 5 - 50 mmoles/l dans la solution prête à l'emploi.

**3.** Procédé selon la revendication 1 ou 2, caractérisé en ce que, comme inhibiteur de l'agrégation plaquettaire, on utilise la chloroquine, l'hydroxychloroquine, la quinacrine ou un mélange d'au moins deux de ces substances.

**4.** Procédé selon au moins l'une des revendications 1 à 3, caractérisé en ce que, comme anticoagulant, on utilise l'hirudine, l'héparine, le dermatane-sulfate, le chondroïtine-sulfate ou un pentosane-polysulfate, à une concentration de 100 - 100 000 unités par litre, ou une association, mais de préférence 5 000 - 50 000 unités d'héparine par litre de la solution prête à l'emploi.

**5.** Procédé selon au moins l'une des revendications 1 a 4, caractérisé en ce que, comme agent chélateur, on utilise un sel de l'acide citrique ou de l'acide oxalique, ou l'EDTA, l'EGTA ou une association de ces substances, à une concentration de 0,2 - 500 mmoles/l, de préférence 2 - 200 mmoles/l d'EDTA.

**6.** Procédé pour la mise en évidence de l'activité fonctionnelle des protéines PP4, PPA-X, PAP III, p68, anchorine et lipocortines I et II, au moyen de partenaires de liaison spécifiques, caractérisé en ce que l'on utilise l'agent préparé selon au moins l'une des revendications 1 à 5.

**7.** Procédé selon la revendication 6, caractérisé en ce que, comme partenaires de liaison spécifiques, on utilise des anticorps, et la concentration des protéines est déterminée par exemple au moyen d'un essai EIA, RIA, CIA ou FIA.

**8.** Procédé selon la revendication 6 ou 7, caractérisé en ce que l'on recueille un échantillon de sang dans un récipient dans lequel est disposé au préalable un volume d'une solution plusieurs fois concentrée de l'agent selon la revendication 1, tel qu'après l'addition de sang la concentration désirée de la substance active de l'agent est atteinte, et on détermine ensuite dans ce mélange, ou dans un échantillon de celui-ci, l'activité fonctionnelle d'annexines.